(19) Europäisches Patentamt
European Patent Office
Office européen des brevets

(11) Veröffentlichungsnummer: **0 021 244**
**B1**

(12) # EUROPÄISCHE PATENTSCHRIFT

(45) Veröffentlichungstag der Patentschrift: **12.01.83**

(51) Int. Cl.³: **C 07 J 41/00,** A 61 K 31/705

(21) Anmeldenummer: **80103244.2**

(22) Anmeldetag: **10.06.80**

(54) **Digoxin-Derivate, Verfahren zu ihrer Herstellung und ihre Verwendung in Arzneimitteln.**

(30) Priorität: **28.06.79 DE 2926026**

(43) Veröffentlichungstag der Anmeldung: **07.01.81 Patentblatt 81/1**

(45) Bekanntmachung des Hinweises auf die Patenterteilung: **12.01.83 Patentblatt 83/2**

(84) Benannte Vertragsstaaten: **AT BE CH DE FR GB IT LI LU NL SE**

(56) Entgegenhaltungen: **FR-A-1 466 165**

(73) Patentinhaber: **BOEHRINGER MANNHEIM GMBH, *Sandhofer Strasse* 112-132 Postfach 31 01 20, D-6800 Mannheim 31-Waldhof (DE)**

(72) Erfinder: **Kaiser, Fritz, Dr.rer.nat., Hans-Holbein-Strasse 10, D-6840 Lampertheim (DE)**
Erfinder: **Koch, Klaus, Dr.rer.nat., Glücksburger Weg 168, D-6800 Mannheim 31 (DE)**
Erfinder: ***Schaumann. Wolfgang, Prof.Dr.med.,* Mönchhofstrasse 58, D-6900 Heidelberg (DE)**
Erfinder: **Voigtländer, Wolfgang, Dr.rer.nat., Haselnussweg 30, D-6940 Weinheim (DE)**

**EP 0 021 244 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Digoxinderivate, Verfahren zu ihrer Herstellung und ihre Verwendung in Arzneimitteln

Gegenstand der vorliegenden Erfindung sind neue 12-Dehydrodigoxinoxime der allgemeinen Formel I

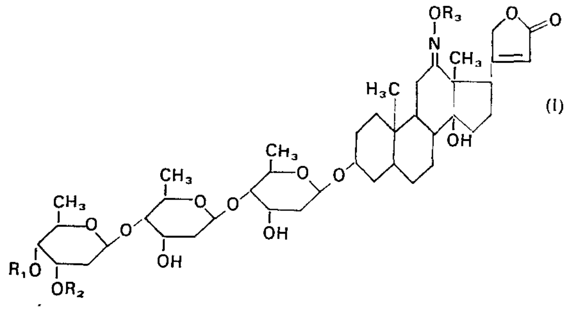

(I)

in der $R_1$ und $R_2$ gleich oder verschieden sind, und Wasserstoff, eine niedere Acyl- oder eine niedere Alkylgruppe darstellen sowie $R_1$ und $R_2$ auch zusammen ein Alkylidenrest mit 2 bis 6 C-Atomen sein können; $R_3$ Wasserstoff oder eine niedere Alkyl- bzw. Aralkylgruppe bedeutet.

Verfahren zu ihrer Herstellung sowie ihre Verwendung zur Herstellung von Arzneimitteln gegen Herzinsuffizienz.

Unter Acylgruppen sind Acylreste mit 1-3 Kohlenstoffatomen zu verstehen, vorzugsweise der Acetylrest.

Als Alkylgruppen kommen Alkylreste mit 1-3 Kohlenstoffatomen in Frage, vorzugsweise die Methylgruppe und mit Aralkylgruppen sind durch einen Phenylrest substituierte Alkylreste mit 1-3 Kohlenstoffatomen, bevorzugt die Benzylgruppe gemeint. Unter Alkylidenrest wird vorzugsweise ein Ethyliden-, Propyliden-1-, Propyliden-2- oder Cyclohexylidenrest verstanden.

Das erfindungsgemässe Verfahren zur Herstellung der Verbindungen der allgemeinen Formel I ist dadurch gekennzeichnet, dass man in an sich bekannter Weise Verbindungen der allgemeinen Formel II

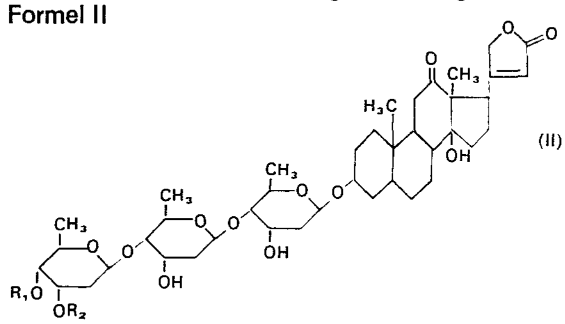

(II)

in der $R_1$ und $R_2$ die vorgenannte Bedeutung haben, gelöst in inerten Lösungsmitteln mit einem Hydroxylaminhydrochlorid der allgemeinen Formel III

$$R_3-O-NH_2 \qquad (III)$$

in der $R_3$ die obige Bedeutung hat,
in Gegenwart einer schwachen Base umsetzt.

Die Ausgangsverbindungen der Formel II sind beispielsweise aus der FR-A Nr. 1466165 bekannte oder in entsprechender Weise herstellbare Substanzen. Die Ausgangsverbindungen zeigen auch cardiotonische Wirkungen, allerdings schwächer als Digoxin, aus dem sie hergestellt werden. Wie Digoxin werden sie fast ausschliesslich über den Harn ausgeschieden, so dass sie diesem gegenüber keine Vorteile bieten und deshalb auch nicht therapeutisch genutzt werden.

Die Verbindungen der Formel II werden bevorzugt in basischen Lösungsmitteln, beispielsweise Dimethylanilin, Triethylamin, Pyridin, gegebenenfalls im Gemisch mit niederen Alkoholen — bevorzugt Ethanol — umgesetzt, wodurch die zusätzliche Base entbehrlich wird. Die Umsetzung findet statt im Bereich von Raum- bis Siedetemperatur des Lösungsmittels bei Reaktionszeiten zwischen 72 h und 1 h.

Die Reindarstellung der Endprodukte erfolgt durch Umkristallisation bzw. chromatographische Trennung oder multiplikative Verteilung mit anschliessender Kristallisation.

Gegebenenfalls kann die Einführung bzw. Umwandlung von Substituenten $R_1$ und $R_2$ in die endständige Digitoxose auch anschliessend an die erfolgte 12-Oximierung nach bekannten Methoden durchgeführt werden.

So wird die Alkylgruppe durch Umsetzung mit einem Diazoalkan, einem Alkylhalogenid oder einem Schwefel- oder Sulfonsäurealkylester, die Acylgruppe durch Umsetzung mit einem aktiven Säurederivat, beispielsweise einem Säurechlorid oder Säureanhydrid und der Alkylidenrest durch Umsetzung mit einem entsprechenden Aldehyd bzw. Keton, vorzugsweise unter Säurekatalyse eingeführt. Die genannten Verbindungen der allgemeinen Formel I sind neu. Sie werden im Gegensatz zu Digoxin und dessen entsprechenden Derivaten überwiegend extrarenal ausgeschieden und bieten damit in der Herzglycosidtherapie, besonders bei Patienten mit verminderter Nierenfunktion, grössere Sicherheit. Sie sind alle lipophiler als Digoxin bzw. ihr entsprechendes Digoxinderivat, werden somit besser resorbiert und besitzen schliesslich eine grössere therapeutische Breite, was sich aus der flachen Dosis/Wirkungskurve bei Injektion steigender Dosen der Wirkstoffe ergibt.

Die pharmakologische Wirkung der erfindungsgemässen Verbindungen wurde durch den Vergleich von

12-Dehydroxydigoxinoxim (Beispiel 1) = BM 62,192

12-Dehydrodigoxin-O-methyloxim (Beispiel 7) = BM 62,211

mit dem als Arzneimittel gegen Herzinsuffizienz bekannten Digoxin in der folgenden Versuchsanordnung bestimmt:

Positiv inotrope und cardiotoxische Wirkung wurden bei Katzen mit Pentobarbital-induzierter Herzinsuffizienz geprüft. Im Abstand von 10 min wurden steigende Dosen injiziert, so dass die Gesamtdosis jeweils um den Faktor $\sqrt{2}$ zunahmen. Als Kriterium für die therapeutisch relevante, positiv inotrope Wirkung diente die Zunahme der maximalen Druckanstiegsgeschwindigkeit um 1,5 bzw. 3,0 mmHg/s ($DE_{1,5}$ und $DE_{3,0}$). Der Quotient aus beiden Grössen ist ein Mass für die Steilheit der Dosiswirkungsgeraden.

Als Mass für die Cardiotoxizität diente die Dosis, bei der die ersten Störungen der Erregungsleitung auftraten. Der Zeitpunkt des Beginns der Arrhythmien wurde aufgrund des EKG festgehalten und durch Interpolation die dazugehörige Dosis errechnet. Der Quotient aus dieser cardiotoxischen Dosis und der $DE_{1,5}$ diente als Mass für die therapeutische Breite.

Wie die Ergebnisse in Tafel 1 zeigen, hatten die Verfahrensprodukte eine grössere therapeutische Breite als Digoxin. Bei BM 62,211 war auch die Dosiswirkungsgerade flacher als bei Digoxin, was ebenfalls als Hinweis auf eine bessere Abstufbarkeit der Wirkung und damit eine leichtere Handhabbarkeit bei der Therapie gewertet werden kann.

Pharmakokinetische Untersuchungen wurden bei wachen Katzen durchgeführt. die Substanzen wurden in $^3H$-markierter Form intravenös injiziert. Während 48 h wurden über einen implantierten Katheter Blutproben zur Bestimmung der Radioaktivität im Plasma entnommen. Der Harn wurde während 14 d gesammelt. Die Eliminationskonstante wurde aus dem Abfall der Plasmakonzentrationen und der im Harn ausgeschiedenen Menge errechnet. Die Mittelwerte der therapeutisch relevanten Parameter sind in Tafel 2 zusammengestellt.

Je niedriger die totale Clearance einer Substanz ist, desto niedriger ist die Tagesdosis, die benötigt wird, um eine bestimmte Plasmakonzentration aufrecht zu erhalten. Die Ergebnisse in Tafel 2 zeigen, dass die Verfahrensprodukte eine deutlich niedrigere totale Clearance hatten. Von BM 62,192 werden rund 45%, von BM 62,211 13% der Dosis von Digoxin benötigt, um dieselben Plasmakonzentrationen zu erreichen.

Für die Steuerbarkeit der Behandlung ist die Geschwindigkeit der Elimination wichtig. Beim Menschen wurde für Digoxin eine Eliminationskonstante von rund 0,015 $h^{-1}$ gefunden, was sehr gut mit den Ergebnissen bei der Katze übereinstimmt. Biologisch gesehen hängt die Eliminationskonstante von der totalen Clearance und dem Verteilungsvolumen ab.

$$\text{Eliminationskonstante} = \frac{\text{totale Clearance}}{\text{Verteilungsvolumen}}$$

Infolge des geringeren Verteilungsvolumens war die Eliminationskonstante von BM 62,211 trotz der geringeren totalen Clearance etwa gleich gross wie die von Digoxin, die von BM 62,192 sogar grösser. Das spricht dafür, dass die therapeutische Wirkung der Verfahrensprodukte selbst dann mindestens so gut steuerbar ist wie die von Digoxin, wenn man von der grösseren therapeutischen Breite absieht.

Ein weiterer vorteil ist die geringere Ausscheidungsquote im Harn. Vergiftungen mit Digoxin treten vor allem bei Patienten mit eingeschränkter Nierenfunktion auf. Da die Verfahrensprodukte zu einem grösseren Teil extrarenal eliminiert werden, ist diese Gefahr geringer.

Tafel 1

Positiv inotrope und cardiotoxische Wirkung von BM 62,192 und BM 62,211 im Vergleich zu Digoxin bei fraktionierter Injektion an Pentobarbital-insuffizienten Katzen

A = Anfangsdosis (mg/kg), Steigerung der Gesamtdosis um den Faktor $\sqrt{2}$ im Abstand von 10 min.

| Substanz | $DE_{1,5}$ (mg/kg) | $DE_{3,0}$ (mg/kg) | $\frac{DE_{3,0}}{DE_{1,5}}$ | Arrh. (mg/kg) | $\frac{\text{Arrh.}}{DE_{1,5}}$ |
|---|---|---|---|---|---|
| Digoxin | 0,120 | 0,253 | 2,28 | 0,330 | 2,75 |
| BM 62,192 | 0,105 | 0,236 | 1,98 | 0,510 | 4,85 |
| BM 62,211 | 0,334 | 0,817 | 3,56 | 1,37 | 4,10 |

Tafel 2

Kinetik von BM 62,192 und BM 62,211 im Vergleich zu Digoxin bei intravenöser Injektion an wachen Katzen

| | Digoxin | BM 62,192 | BM 62,211 |
|---|---|---|---|
| Dosis (µg/kg) | 30 | 100 | 100 |
| Verteilungsvolumen (l/kg) | 13,3 | 2,74 | 1,47 |
| Eliminationskonstante ($h^{-1}$) | 0,01214 | 0,0258 | 0,0143 |
| Renale Clearance (ml/kg·min) | 1,24 | 0,32 | 0,12 |
| Totale Clearance (ml/kg·min) | 2,68 | 1,19 | 0,35 |
| Ausscheidung in Harn (% der Dosis) | 42,8 | 30 | 31 |

Die erfindungsgemässen neuen Substanzen I können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler und Puffer enthält. Derartige Zusätze sind z.B. Tartrat- und Citratpuffer, Ethanol, Komplexbildner (wie Ethylendiamintetraessigsäure und deren nichttoxische Salze), hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, höher-molekulare Fettsäuren (wie Stearinsäure), Gelatine, Agar-Agar, Calciumphosphat, Magnesiumstearat, tierische und pflanzliche Fette, feste hoch-molekulare Polymere (wie Polyethylenglykole); für orale Applikation geeignete Zubereitungen können gewünschtenfalls Geschmacks- und Süssstoffe enthalten. Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 0,05-1,0 mg für Warmblüter mit einem Gewicht von etwa 70 kg.

In den nachfolgenden Beispielen ist die Herstellung dieser neuen Verbindungen erläutert.

Dünnschichtchromatogramme:

DC-Fertigplatten Merck, Kieselgel 60/F 254

DC-Fliessmittel I: Xylol/Methylethylketon 2:3 +5% Formamid. Imprägnierung 20% Formamid in Aceton

DC-Fliessmittel II: Chloroform/Methylethylketon 1:3

Detektion: Trichloressigsäure/Chloramin-Reagens Fluoreszenzen im langwelligen UV ($\lambda$ = 360 nm)

$R_D$ bedeutet den auf die Laufstrecke von Digoxin bezogenen R-Wert

$$\left(\text{Beispiel: } \frac{\text{Laufstrecke 12-Dehydrodigoxinoxim}}{\text{Laufstrecke Digoxin}}\right)$$

$R_{DD}$ bedeutet den auf die Laufstrecke des entsprechenden Digoxinderivates bezogenen R-Wert

$$\left(\text{Beispiel: } \frac{\text{Laufstrecke 12-Dehydro-}\beta\text{-methyldigoxinoxim}}{\text{Laufstrecke }\beta\text{-Methyldigoxin}}\right)$$

*Beispiel 1*

*12-Dehydrodigoxinoxim*

3 g 12-Dehydrodigoxin, in 60 ml Pyridin (wasserfrei) und 60 ml Ethanol (wasserfrei) gelöst, werden nach Zugabe von 600 mg Hydroxylaminhydrochlorid 6 h unter Rückfluss zum Sieden erhitzt. Anschliessend wird mit 1,2 l Wasser verdünnt, mit Chloroform (6mal 1/10 Volumen) ausgeschüttelt, die Chloroformphasen mit 2 N-Schwefelsäure, Sodalösung und Wasser gewaschen, über Natriumsulfat getrocknet und im Vakuum eingeengt. Der Rückstand liefert nach Kristallisation aus Chloroform/Ether/Petroether 2,5 g 12-Dehydrodigoxinoxim.

Schmelzpunkt: 172-176°C

DC-Fliessmittel I: $R_D$ = 2,0

DC-Fliessmittel II: $R_D$ = 1,28

*Beispiel 2*

*12-Dehydro-α-methyldigoxinoxim*

1 g 12-Dehydro-α-methyldigoxin, in 10 ml Pyridin (wasserfrei) und 20 ml Ethanol (wasserfrei) gelöst, wird nach Zugabe von 500 mg Hydroxylaminhydrochlorid 2 h unter Rückfluss zum Sieden erhitzt und, wie unter Beispiel 1 beschrieben, aufgearbeitet. Das Rohprodukt wird mit Cyclohexan/Essigester 2:3 über eine Cellulosesäule (mit Formamid imprägniert) getrennt. Die chromatographisch einheitlichen Fraktionen liefern nach Kristallisation aus Chloroform/Ether/Petrolether 460 mg 12-Dehydro-α-methyldigoxinoxim.

Schmelzpunkt: 162-166°C

DC-Fliessmittel I: $R_D$ = 2,79, $R_{DD}$ = 1,33

DC-Fliessmittel II: $R_D$ = 1,96, $R_{DD}$ = 1,23

*Beispiel 3*

*12-Dehydro-β-methyldigoxinoxim*

3 g 12-Dehydro-β-methyldigoxin, in 60 ml Pyridin (wasserfrei) und 60 ml Ethanol (wasserfrei) gelöst, werden nach Zugabe von 600 mg Hydroxylaminhydrochlorid, wie unter Beispiel 1 beschrieben, umgesetzt und aufgearbeitet. Das Rohprodukt wird mit Cyclohexan/Essigester 1:1 über eine Cellulosesäule (mit Formamid imprägniert) getrennt. Die chromatographisch einheitlichen Fraktionen ergeben nach Kristallisation aus Chloroform/Ether/Petrolether 2,2 g 12-Dehydro-β-methyldigoxinoxim.

Schmelzpunkt: 151-154°C

DC-Fliessmittel I: $R_D$ = 2,84, $R_{DD}$ = 1,28

DC-Fliessmittel II: $R_D$ = 2,03, $R_{DD}$ = 1,22

*Beispiel 4*

*12-Dehydro-3''', 4'''-isopropylidendigoxinoxim*

1 g 12-Dehydro-3''', 4'''-isopropylidendigoxin, in 10 ml Pyridin (wasserfrei) und 20 ml Ethanol (wasserfrei) gelöst, wird nach Zugabe von 500 mg Hydroxylaminhydrochlorid, wie unter Beispiel 2 beschrieben, umgesetzt und aufgearbeitet. Das Rohprodukt wird mit Cyclohexan/Essigester 3:1 über eine Cellulosesäule (mit Formamid imprägniert) getrennt. Die chromatographisch einheitlichen Fraktionen liefern nach Kristallisation aus Chloroform/Ether/Petrolether 430 mg 12-Dehydro-3''', 4'''-isopropylidendigoxinoxim.

Schmelzpunkt: 234-237°C

DC-Fliessmittel I: $R_D$ = 3,57, $R_{DD}$ = 1,06

DC-Fliessmittel II: $R_D$ = 2,89, $R_{DD}$ = 1,16

*Beispiel 5*

*12-Dehydro-α-acetyldigoxinoxim*

1 g 12-Dehydrodigoxinoxim, in 20 ml Tetrahydrofuran (wasserfrei) gelöst, wird nach Zugabe von 20 ml Orthoessigsäuretriethylester und 2 g Zinkchlorid (wasserfrei) 6 h bei Raumtemperatur gerührt. Anschliessend wird mit 20 ml Wasser versetzt, 20 h bei Raumtemperatur stehen gelassen, mit 200 ml Wasser verdünnt und mit Chloroform (6mal 1/10 Volumen) ausgeschüttelt. Die Chloroformphasen werden mit Wasser gewaschen, über Natriumsulfat getrocknet und im Vakuum eingeengt. Der Rückstand wird mit Cyclohexan/Essigester 2:3 über eine Cellulosesäule (mit Formamid imprägniert) getrennt. Die chromatographisch einheitlichen Fraktionen ergeben nach Kristallisation aus Chloroform/Ether 350 mg 12-Dehydro-α-acetyldigoxinoxim.

Schmelzpunkt: 188-192°C

DC-Fliessmittel I: $R_D$ = 2,79, $R_{DD}$ = 1,38

DC-Fliessmittel II: $R_D$ = 2,0 , $R_{DD}$ = 1,25

*Beispiel 6*

*12-Dehydro-β-acetyldigoxinoxim*

1 g 12-Dehydrodigoxinoxim, in 10 ml Dimethylformamid gelöst, wird nach Zugabe von 200 mg Triethylendiamin und 140 g Essigsäureanhydrid 24 h bei Raumtemperatur stehen gelassen. Anschliessend wird mit 100 ml Wasser verdünnt, mit

Chloroform ausgeschüttelt und die Chloroformphasen, nach Waschen mit 2N-Schwefelsäure, Sodalösung und Vasser, im Vakuum eingeengt. Das Rohprodukt wird mit Cyclohexan/Essigester 2:3 über eine Cellulosesäule (mit Formamid imprägniert) getrennt. Die chromatographisch einheitlichen Fraktionen liefern nach Kristallisation aus Chloroform/Ether/Petrolether 220 mg 12-Dehydro-β-acetyldigoxinoxim.
Schmelzpunkt: 161-164°C
DC-Fliessmittel I: $R_D$ = 2,84, $R_{DD}$ = 1,23
DC-Fliessmittel II: $R_D$ = 2,27, $R_{DD}$ = 1,19

*Beispiel 7*

*12-Dehydrodigoxin-O-methyloxim*

3 g 12-Dehydrodigoxin, in 30 ml Pyridin gelöst, werden nach Zugabe von 1,5 g O-Methylhydroxylaminhydrochlorid 72 h bei Raumtemperatur stehen gelassen. Anschliessend wird mit 1 l Wasser verdünnt, mit Chloroform (6mal 1/10 Volumen) ausgeschüttelt, die Chloroformphase mit 2N-Schwefelsäure, Sodalösung und Wasser gewaschen, über Natriumsulfat getrocknet und im Vakuum eingeengt. Der Rückstand liefert nach Kristallisation aus Essigester/Ether 2,85 g 12-Dehydrodigoxin-O-methyloxim.
Schmelzpunkt: 221-225°C
DC-Fliessmittel I: $R_D$ = 3,84
DC-Fliessmittel II: $R_D$ = 1,44

*Beispiel 8*

*12-Dehydrodigoxin-O-ethyloxim*

3 g 12-Dehydrodigoxin, in 30 ml Pyridin und 60 ml Ethanol (wasserfrei) gelöst, werden nach Zugabe von 1,5 g O-Ethylhydroxylaminhydrochlorid, wie unter Beispiel 1 beschrieben, umgesetzt und aufgearbeitet. Das Rohprodukt wird mit Cyclohexan/Essigester 2:1 über eine Cellulosesäule (mit Formamid imprägniert) getrennt. Die chromatographisch einheitlichen Fraktionen liefern nach Kristallisation aus Aceton/Ether 720 mg 12-Dehydrodigoxin-O-ethyloxim.
Schmelzpunkt: 200-204°C
DC-Fliessmittel I: $R_D$ = 4,08
DC-Fliessmittel II: $R_D$ = 1,60

*Beispiel 9*

*12-Dehydrodigoxin-O-benzyloxim*

3 g 12-Dehydrodigoxin, in 30 ml Pyridin und 60 ml Ethanol (wasserfrei) gelöst, werden nach Zugabe von 1,5 g O-Benzylhydroxylaminhydrochlorid, wie unter Beispiel 1 beschrieben, umgesetzt und aufgearbeitet. Das Rohprodukt wird mit Cyclohexan/Essigester 2:1 über Cellulosesäule (mit Formamid imprägniert) getrennt. Die chromatographisch einheitlichen Fraktionen liefern nach Kristallisation aus Aceton/Ether 390 mg 12-Dehydrodigoxin-O-benzyloxim.
Schmelzpunkt: 222-225°C
DC-Fliessmittel I: $R_D$ = 4,28
DC-Fliessmittel II: $R_D$ = 1,57

*Beispiel 10*

*12-Dehydro-β-acetyldigoxin-O-methyloxim*

3 g 12-Dehydrodigoxin, in 30 ml Pyridin gelöst, werden nach Zugabe von 1,5 g O-Methylhydroxylaminhydrochlorid, wie unter Beispiel 7 beschrieben, umgesetzt und aufgearbeitet. Das Rohprodukt (3,1 g) wird in 31 ml Dimethylformamid gelöst, nach Zugabe von 630 mg Triethylendiamin und 720 mg Essigsäureanhydrid, wie unter Beispiel 6 beschrieben, umgesetzt und aufgearbeitet. Das Rohprodukt liefert nach Kristallisation aus Essigester/Ether 2,7 g 12-Dehydro-β-acetyldigoxin-O-methyloxim.
Schmelzpunkt: 209-212°C
DC-Fliessmittel I: $R_D$ = 4,51, $R_{DD}$ = 1,88
DC-Fliessmittel II: $R_D$ = 2,24, $R_{DD}$ = 1,28

*Beispiel 11*

*12-Dehydro-β-methyldigoxin-O-methyloxim*

3 g 12-Dehydro-β-methyldigoxin, in 30 ml Pyridin gelöst, werden nach Zugabe von 1,5 g O-Methylhydroxylaminhydrochlorid, wie unter Beispiel 7 beschrieben, umgesetzt und aufgearbeitet. Das Rohprodukt (3,35 g) kommt zur multiplikativen Verteilung mit dem Phasengemisch Tetrachlorkohlenstoff/Essigester/Methanol/Wasser 15:1:12:4. Die wässrige Phase liefert nach Einengen und Kristallisation aus Essigester/Ether 2,2 g 12-Dehydro-β-methyldigoxin-O-methyloxim.
Schmelzpunkt: 214-218°C
DC-Fliessmittel I: $R_D$ = 3,66, $R_{DD}$ = 1,52
DC-Fliessmittel II: $R_D$ = 1,45, $R_{DD}$ = 0,93

**Patentansprüche**

1. 12-Dehydrodigoxinoxime der allgemeinen Formel I

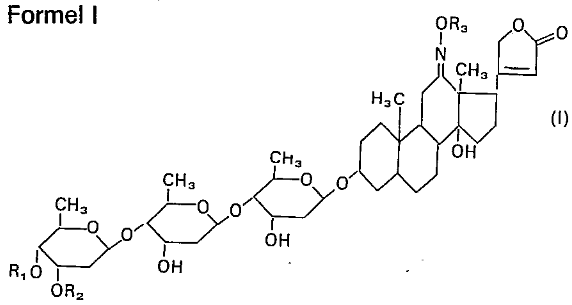

(I)

in der $R_1$ und $R_2$ gleich oder verschieden sind, und Wasserstoff, eine niedere Acyl- oder eine niedere Alkylgruppe darstellen sowie $R_1$ und $R_2$ auch zusammen ein Alkylidenrest mit 2 bis 6 C-Atomen sein können; $R_3$ Wasserstoff oder eine niedere alkyl- bzw. Aralkyl-Gruppe bedeutet.

2. Verfahren zur Herstellung von 12-Dehydrodigoxinoximen der allgemeinen Formel I

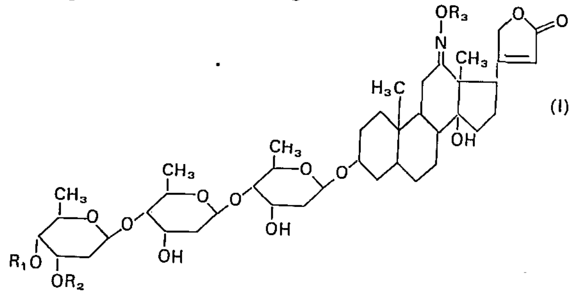

(I)

in der $R_1$ und $R_2$ gleich oder verschieden sind, und Wasserstoff, eine niedere Acyl- oder eine niedere Alkylgruppe darstellen sowie $R_1$ und $R_2$ auch zusammen ein Alkylidenrest mit 2 bis 6 C-Atomen sein können; $R_3$ Wasserstoff oder eine niedere Alkyl- bzw. Aralkylgruppe bedeutet, dadurch gekennzeichnet, dass man in an sich bekannter Weise Verbindungen der allgemeinen Formel II

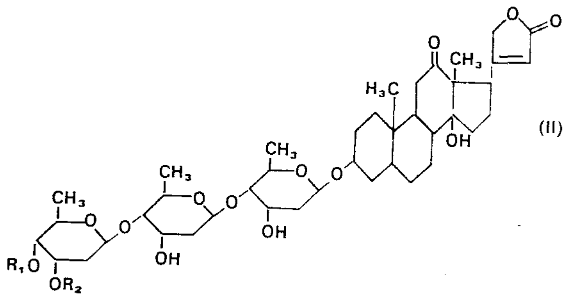

(II)

in der $R_1$ und $R_2$ die vorgenannte Bedeutung haben, gelöst in inerten Lösungsmitteln mit einem Hydroxylaminhydrochlorid der Formel III

$$R_3-O-NH_2 \qquad (III)$$

in der $R_3$ die obige Bedeutung hat, in Gegenwart einer schwachen Base umsetzt, wobei die Reste $R_1$ und $R_2$ auch nachträglich eingeführt bzw. umgewandelt werden können

3. Verwendung von 12-Dehydrodigoxinoximen der allgemeinen Formel I

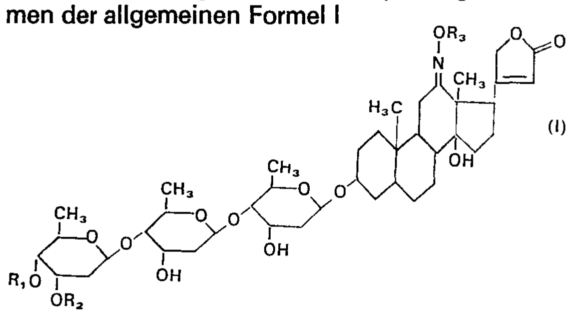

(I)

in der $R_1$ und $R_2$ gleich oder verschieden sind, und Wasserstoff, eine niedere Acyl- oder eine niedere Alkylgruppe darstellen sowie $R_1$ und $R_2$ auch zusammen ein Alkylidenrest mit 2 bis 6 C-Atomen sein können; $R_3$ Wasserstoff oder eine niedere Alkyl- bzw. Aralkylgruppe bedeutet, zur Herstellung von Arzneimitteln gegen Herzinsuffizienz.

4. Arzneimittel, gekennzeichnet durch einen Gehalt an 12-Dehydrodigoxinoxime der allgemeinen Formel I

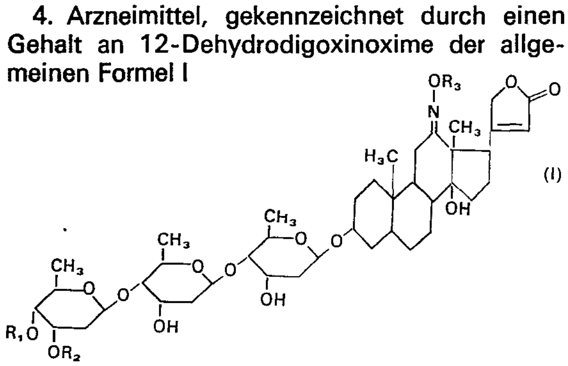

(I)

in der $R_1$ und $R_2$ gleich oder verschieden sind, und Wasserstoff, eine niedere Acyl- oder eine niedere Alkylgruppe darstellen sowie $R_1$ und $R_2$ auch zusammen ein Alkylidenrest mit 2 bis 6 C-Atomen sein können; $R_3$ Wasserstoff oder eine niedere Alkyl- bzw. Aralkylgruppe bedeutet.

## Claims

1. 12-Dehydrodigoxin oximes of the general formula I

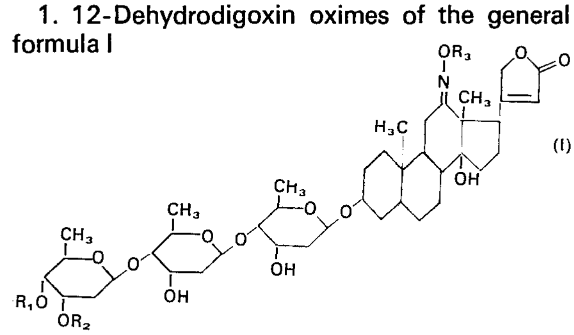

(I)

in which $R_1$ and $R_2$ are the same or different, and represent hydrogen, a lower acyl or a lower alkyl groupe, as well as $R_1$ and $R_2$ together can be an alkylidene radical with 2 to 6 C-atoms; $R_3$ signifies hydrogen or a lower alkyl or aralkyl group.

2. Process for the preparation of 12-dehydrodigoxin oximes of the general formula I

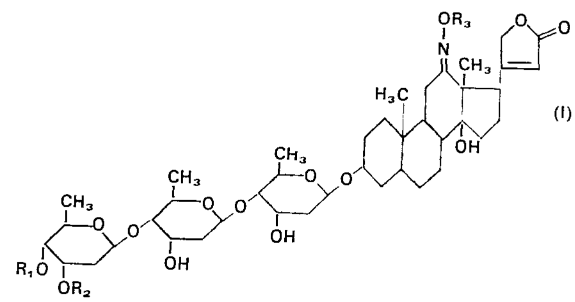

(I)

in which $R_1$ and $R_2$ are the same or different, and represent hydrogen, a lower acyl or a lower alkyl group, as well as $R_1$ and $R_2$ together can be an alkylidene radical with 2 to 6 C-atoms; $R_3$ signifies hydrogen or a lower alkyl or aralkyl group, characterized in that, in per se known manner, one reacts a compound of the general formula II

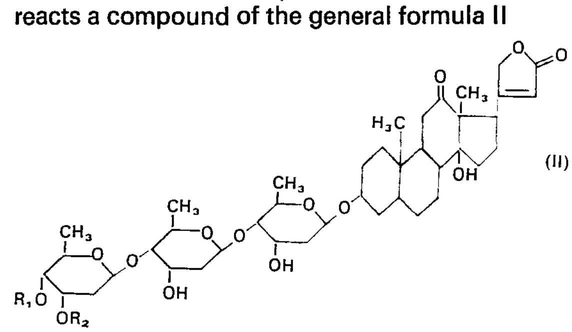

(II)

in which $R_1$ and $R_2$ have the previously mentioned meaning, dissolved in an inert solvent, with a hydroxylamine hydrochloride of the formula III

$$R_3-O-NH_2 \qquad (III)$$

in which $R_3$ has the above meaning, in the presence of a weak base, whereby the residues $R_1$ and $R_2$ can also be subsequently introduced or changed.

3. Use of 12-dehydrodigoxin oximes of the general formula I

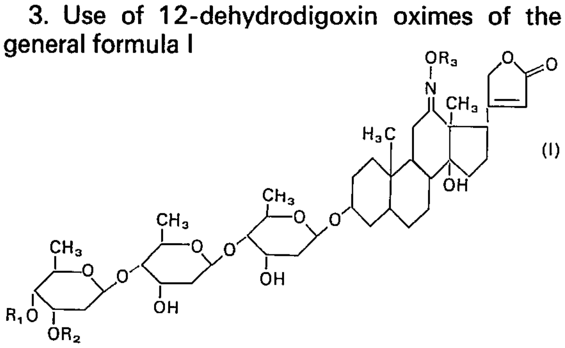

(I)

in which $R_1$ and $R_2$ are the same or different, and represent hydrogen, a lower acyl or a lower alkyl group, as well as $R_1$ and $R_2$ together can be an alkylidene radical with 2 to 6 C-atoms; $R_3$ signifies hydrogen or a lower alkyl or aralkyl group,
for the preparation of medicaments against heart insufficiency.

4. Medicaments, characterized by a content of 12-dehydrodigoxin oximes of the general formula I

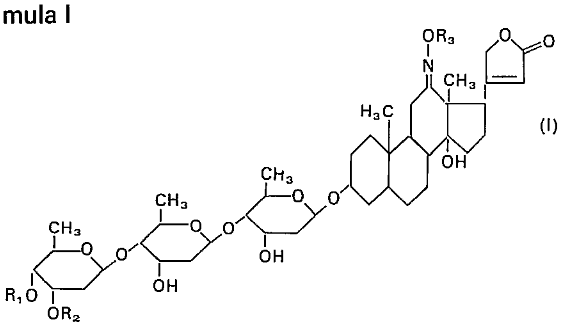

(I)

in which $R_1$ and $R_2$ are the same or different, and represent hydrogen, a lower acyl or a lower alkyl group, as well as $R_1$ and $R_2$ together can be an alkylidene radical with 2 to 6 C-atoms; $R_3$ signifies hydrogen or a lower alkyl or aralkyl group.

## Revendications

1. Dérivés de 12-déhydrodigoxinoxime de formule générale I

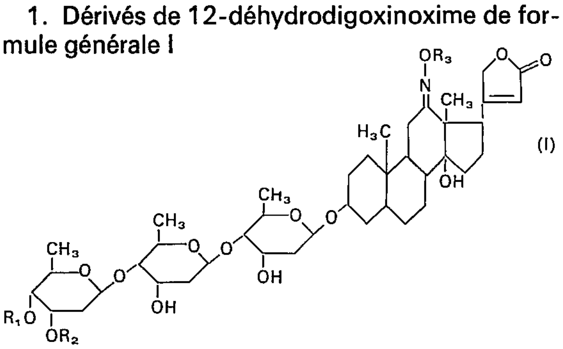

(I)

dans laquelle $R_1$ et $R_2$ peuvent être identiques ou différents, et désignent un atome d'hydrogène, un groupe acyle inférieur ou un groupe alkyle inférieur, et $R_1$ et $R_2$ peuvent former aussi ensemble un reste alkylidène ayant de 2 à 6 atomes de carbone; $R_3$ est un atome d'hydrogène ou un groupe alkyle ou aralkyle inférieur.

2. Procédé pour la préparation de dérivés de 12-déhydrodigoxinoxime de formule générale I

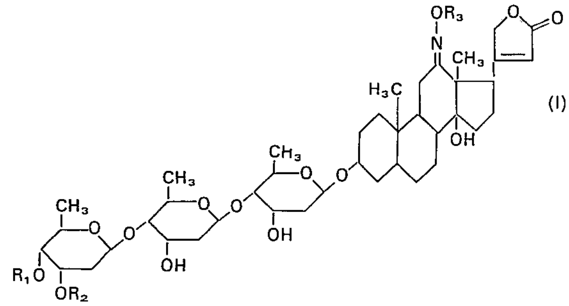

(I)

dans laquelle $R_1$ et $R_2$ sont identiques ou différents, et désignent un atome d'hydrogène, un groupe acyle ou alkyle inférieur, et $R_1$ et $R_2$ peuvent aussi former ensemble un reste alkylidène ayant de 2 à 6 atomes de carbone; $R_3$ est de l'hydrogène ou un groupe alkyle ou aralkyle inférieur, caractérisé en ce que, de façon en soi connue, on fait réagir des composés de formule générale II

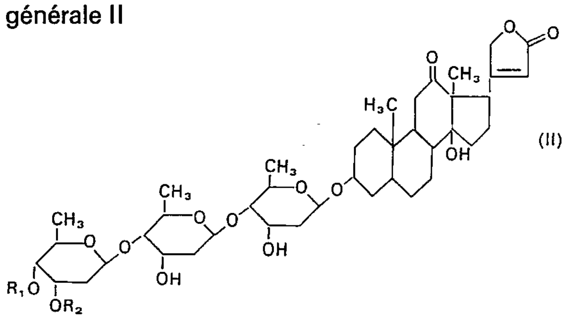

(II)

dans laquelle $R_1$ et $R_2$ ont la signification ci-dessus indiquée, sous forme de solution dans un solvant inerte avec un chlorhydrate d'hydroxylamine de formule III

$$R_3{-}O{-}NH_2 \qquad (III)$$

dans laquelle $R_3$ a la signification indiquée ci-dessus, en présence d'une base faible, les restes $R_1$ et $R_2$ pouvant aussi être introduits ou transformés après cette réaction.

3. Utilisation de dérivés de 12-déhydrodigoxinoxime de formule générale I

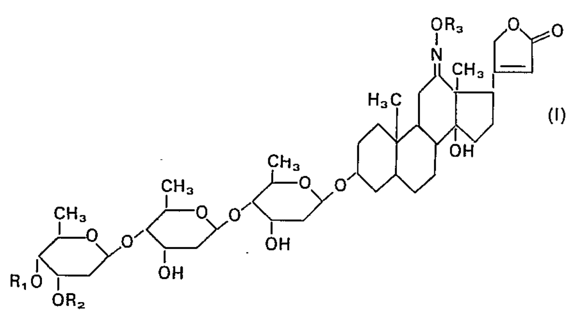

(I)

dans laquelle $R_1$ et $R_2$ sont identiques ou différents, et désignent un groupe acyle ou alkyle inférieur, et $R_1$ et $R_2$ peuvent aussi former ensemble un reste alkylidène ayant de 2 à 6 atomes de carbone; $R_3$ est de l'hydrogène ou un groupe alkyle ou aralkyle inférieur,
pour la fabrication de médicaments contre l'insuffisance cardiaque.

4. Médicaments caractérisés en ce qu'ils ont

une teneur en dérivés de 12-déhydrodigoxinoxime de formule générale I

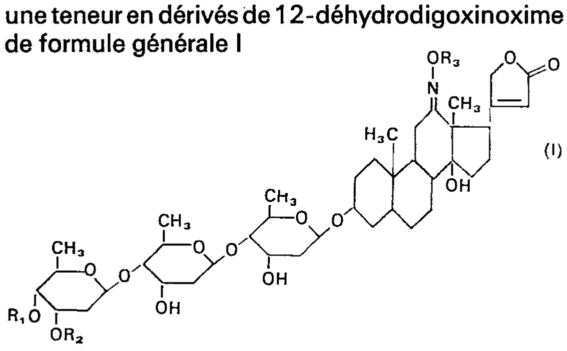

(I)

dans laquelle $R_1$ et $R_2$ sont identiques ou différents, et désignent un atome d'hydrogène, un groupe acyle ou alkyle inférieur, et $R_1$ et $R_2$ peuvent aussi former ensemble un reste alkylidène ayant de 2 à 6 atomes de carbone; $R_3$ est de l'hydrogène ou un groupe alkyle ou aralkyle inférieur.